## Europäisches Patentamt

## European Patent Office

## Office européen des brevets

(11) Publication number: **0 060 683**
**B1**

(12) **EUROPEAN PATENT SPECIFICATION**

(45) Date of publication of patent specification: **26.09.90**

(51) Int. Cl.⁵: **H 05 F 3/02, H 01 B 7/06**

(21) Application number: **82301231.5**

(22) Date of filing: **11.03.82**

(54) Static electricity dissipating device.

(30) Priority: **17.03.81 US 244617**

(43) Date of publication of application:
**22.09.82 Bulletin 82/38**

(45) Publication of the grant of the patent:
**26.09.90 Bulletin 90/39**

(84) Designated Contracting States:
**DE FR GB IT SE**

(56) References cited:
**DE-A-2 543 811**
**DE-A-2 547 390**
**US-A-2 712 098**
**US-A-3 015 754**
**US-A-4 215 384**

(73) Proprietor: **HONEYWELL INC.**
**Honeywell Plaza**
**Minneapolis Minnesota 55408 (US)**

(72) Inventor: **Mykkanen, Fred C.**
**6840 Jefferson N.E.**
**Fridley Minnesota 55432 (US)**

(74) Representative: **Fox-Male, Nicholas Vincent Humbert**
**Honeywell Control Systems Limited Charles Square**
**Bracknell Berkshire RG12 1EB (GB)**

Courier Press, Leamington Spa, England.

## Description

This invention relates generally to devices used to prevent damage to electrostatic sensitive equipment; and more particularly it concerns personnel grounding, so-called 'wrist straps" useful in such applications.

The problem of preventing damage to electron-static sensitive equipment is a serious one. As an example, in fabrication of electronic as well as other apparatus employing plastics parts, the problem of unwanted and destructive static build-up and arcing, which can lead to damged electronic equipment, shocks to employees and fires, is acute. In the past, grounding wires have been used to conduct electrostatic charge build-up on personnel handling electronic parts to grounded members, and for this purpose wrist straps were attached to the arms of users to conduct charge to the grounding wires The precharacterising part of claim 1 is known from DE—A—2547390. Problems encountered included inadvertent wire disconnection from the grounding member and from the wrist strap, as when a worker moves his arm as a way to tension the wire, and wire breakage. One expedient to counter this problem was to wind the wire around the wrist strap, or to tape the wire; however, this was found objectionable, one reason being that it interfered with good electrical contact between the wrist strap and the user's wrist. The cost of equipment damage and reduced worker efficiency due to such static build-up is extremely high.

It is an aim of the invention to provide a simple device which does not interfere with a worker's tasks, and which removes or substantially reduces the static build-up problem.

According to the invention, there is provided a safety device connectible between a human and an electrically conductive terminal, the device comprising a first electrically conducting part connectible to said terminal, a second electrically conducting part connectible to said human, and flexible means electrically connecting together said parts, said flexible means including in electrical series connection a first electrical cable, a protective electrical resistor connector and an elongate flexible connection member, characterised by a releasable plug and socket connector connected in electrical series between said electrical resistor and said elongate flexible connection member and by a second electrical cable electrically connected to said first part and one of said plug or socket and being of length longer than said flexible member so that when the flexible member is under tension the second cable is tension free.

An embodiment of the present invention will now be described by way of example only, with reference to the accompanying drawings, in which:-

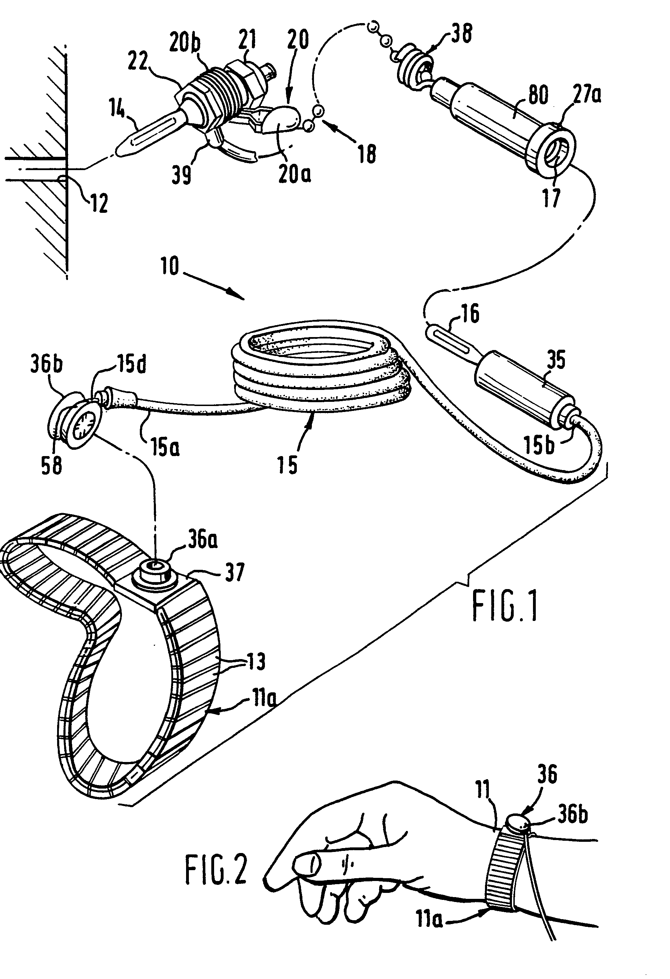

Figure 1 is a perspective view of a static electricity dissipating device according to the invention,

Figure 2 is an elevation showing the manner in which the Figure 1 apparatus may be connected to the wrist of a user; and

Figure 3 is a fragmentary view showing details of connections that may be employed in the Figure 1 apparatus.

In the drawings, the device 10 is connectible between the wrist 11 of the user and an electrically conductive terminal or grounding terminal 12. At one end of the device, a conductive metal wrist strap 11a is well adapted to be easily emplaced on the wrist 11, for establishing good electrical contact therewith. The strap for example is expansible and comprises interconnected metallic links 13. At the opposite end of the device, a conductive part such as "banana" plug 14 is well adapted to be easily and removably inserted into terminal 12, to establish good electrical contact with same.

The device 10 includes, between strap 11a and plug 14, a first electrical cable, as for example insulated cable 15, having one end 15a adapted for electrical connection to the user via strap 11a and means interconnecting the opposite end 15b of the cable and the plug 14. Such means includes releasably interconnected plug and socket elements as at 16 and 17, and an elongated flexible connection 18 located between the elements 16 and 17 and the plug 14. The interconnected elements 16 and 17 are quickly releasable (as by relative separation) in response to endwise tension exertion transmitted between the cable 15 and the flexible connection 18, as for example when a user's wrist or arm is for some reason displaced sufficiently away from the grounding terminal 12. Such separation can occur in response to tension exertion in any direction due to the flexibility of connection 18 and the flexibility of the cable 15, respectively extending endwise oppositely of the interconnected elements 16 and 17. This is to be contrasted with the relative insensitivity to tension exertion of plug 14 and fixed position terminal 12, their relative separation only occuring in response to tension exertion in one axial direction.

More specifically, the flexible connection 18 advantageously comprises an electrically conductive, metal chain of interconnected link members such as beads as shown. One end 18a of the chain is swival connected to the plug 14, as via a metallic part or coupler 20. The latter includes a receptacle 20a for the end bead 18a of the chain, and an apertured portion 20b integral with receptacle 20a and loosely swiveled on an endwise extension 14a of the plug 14, extension 14a being threaded to receive stop nut 21. Located between stop nut 21 and flange 22 on the plug are two washers 23 and 24, the bead chain coupler end 20b being loosely confined between such parts. Also threaded on extension 14a is a nut 24a. Accordingly, the worker-user is enabled to move his hands and arma freely, and the chain 18 will swivel about the axis 25 of plug 14 in response to such movement, without pulling the plug 14 from its grounding terminal 12. Any static electricity carried by the user is displaced via the device to the terminal 12.

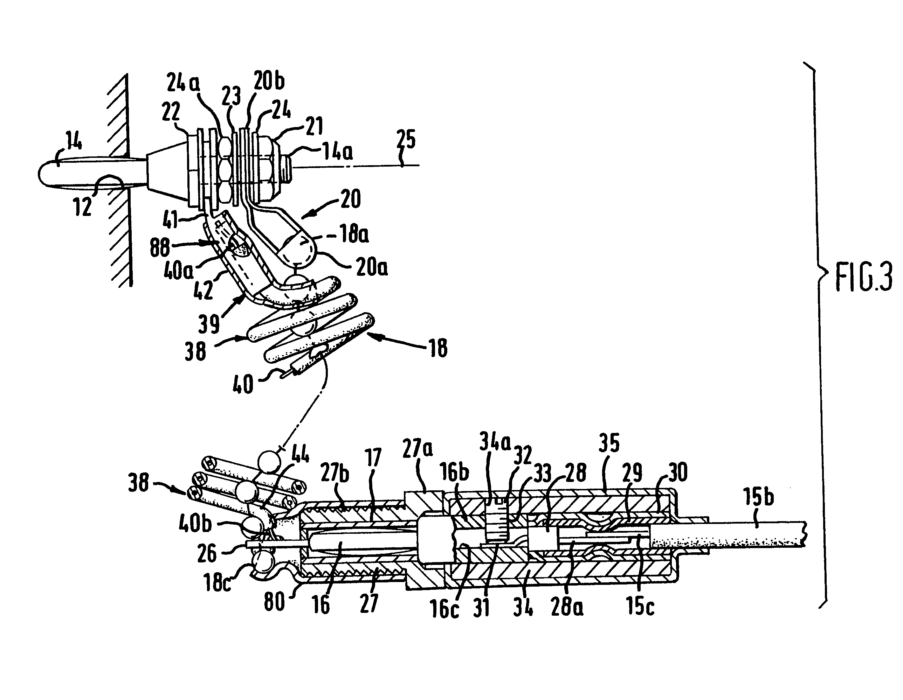

The opposite end bead 18c of the chain is shown in Figure 3 as soldered to a metallic post 26 integral with metallic socket element 17. Surrounding the latter is an electrically insulating sleeve or housing 27 of moulded plastics, having an end flange 27a. Sleeve 27 may be externally grooved or threaded at 27b to accept and retain a length of protective plastics tubing 80 having a shrink fit over the sleeve and over the soldered connection of the bead 18c to post 26, as shown.

The plug 16 has electrical connection with an electrical wire 15c projecting from the end of cable 15, as shown in Figure 3, and typically via a protective electrical resistor 28, connected in series between 16 and 15c. The resistor protects against excessive current flow between elements 12 and 11a, (should, for example, the wearer touch a high power source). A crimped sleeve 29 is employed to connect wire 15c to wire 28a of the resistor, and a molded plastics sheath 30 is fitted over the sleeve 29 and the resistor cylinder. The opposite end resistor wire 31 is shown as forcibly biased against the inner surface 16c of the plug extension 16b by a set screw 32 having threaded interconnection at 33 with the extension 16b. A molded plastics tubular housing 34 fits over the described elements, and has a side opening 34a receiving the head of the screw 32. A length of shrinkable plastics tubing 35 fits over the assembly.

The opposite end 15a of the cable 15 is connected with the wrist strap 11a as via a snap connection 36 which includes a metallic male snap part 36a integral with plate 37 of strip 11c, and a female part 36b having a radially crimped connection 58 to an electrical wire 15d protruding from the insulative sheath of the cable. Release of the snap connection allows the worker-user to move about, free of the apparatus excepting for the wrist band.

A second electrical cable 38 is connected between one of the releasable elements 16 and 17 and the part or plug 14, to transmit electrical current between them, the cable 38 being longer than chain 18 so that it will remain in a slack condition, i.e. free of tension when the bead chain 18 is tensioned. As shown, the second cable 38 advantageously is loosely coiled about the bead chain, and includes an outer electrically insulating sheath 39 covering an electrical wire 40. One end 40a of the wire is held clamped to the fitting 41 by crimping of terminal 88. Fitting 41 contains an opening to fit over and connect with the plug extension 14a, nut 24a clamping fitting 41 against flange 22 to provide positive electrical continuity. Coiled cable 38 flexes to accommodate swiveling of bead chain washer 20b. Terminal 88 affords both mechanical strain relief and electrical connection. The bending of part 20 beyond the stop nut 21 is arranged so that the bead chain and cable 38 may swivel free of interference with the nut 21. Electrically insulating flexible tubing length 42 extends about the connection between wire end 40a and fitting 41.

The opposite end 40b of the wire 40 is soldered to the post 26 near the end bead 18c, and a length of electrically insulating heat shrink sleeving 80 is shrunk over the connection as shown. Accordingly, a dual or parallel (redundant) electrical connection is made between plug 14 and socket 17, via flexible elements 18 and 38, at least one of the latter being tension transmitting, ensuring that electrical continuity is maintained, which enables release of the plug 16 from socket 17 in response to sufficient tension transmission (9 to 22N). This provides a sensory feedback to the wearer indicating inadvertent disconnection.

The above described device has the following additional advantages: it is comfortable to the wearer, attractive in appearance, and possesses electrically and mechanical integrity, and is non-hazardous. For example, the coiled cable 38 insulates the metallic bead chain 18 against contact with voltage sources. The swival connections prevent sharp bends in the wire assembly in the event angular pull forces are applied. The devic prevents accumulation of static charge on the body, while allowing freedom of movement, protection aginst dangerous electrical currents, and enables three possible quick break-away zones in emergencies for operator protection.

**Claims**

1. A safety device connectible between a human and an electrically conductive terminal, the device comprising a first electrically conducting part (14) connectible to said terminal (12), a second electrically conductor part (11a) connectable to said human, and flexible means electrically connecting together said parts (14, 11a), said flexible means including in electrical series connection a first electrical cable (15), a protective electrical resistor (28) and an elongate flexible member (18), characterized by a releasable plug and socket connector (16, 17) connected in electrical series between said electrical resistor (28) and said elongate flexible connection member (18) and by a second electrical cable (38) electrially connected to said first part (14) and one of said plug or socket (16, 17) and being of length longer than said flexible connection member so that when the flexible member is under tension the second cable is tension free.

2. The device of Claim 1, wherein the second cable (38) is coiled about the flexible member (18).

The device of Claim 1 or 2, wherein the flexible member (18) is a metal chain of interconnected beads or other link members.

4. The device of Claim 1, 2 or 3, wherein said first part is a plug (14) axially insertable into a terminal socket (12), said plug having an axial extension (14a) on which the flexible member (18 is mounted for relative swivelling movement.

5. The device of any one of the preceding claims, wherein the second part (11a) is in the form of a strap, and wherein said flexible means is releasably attached to the second part by means of snap connector (36a, 36b) carried by the strap and first mentioned electric cable.

## Patentansprüche

1. Sicherheitsvorrichtung für den Anschluß zwischen einem Menschen und einer stromführenden Klemme, bestehend aus einem ersten elektrisch leitendem Teil (14) für den Anschluß an besagte Klemme (12), einem zweiten elektrisch leitenden Teil (11a) für den Anschluß an besagten Menschen und ein flexibles Mittel für die elektrische Verbindung der besagten Teile (14, 11a), wobei besagtes flexibles Mittel in elektrischer Reihenschaltung ein erstes elektrisches Kabel (15), einen elektrischen Schutzwiderstand (28) und ein langgezogenes flexibles Glied (18) beinhaltet, dadurch gekennzeichnet, daß eine lösbare Steckverbindung (16, 17) zwischen besagtem elektrischen Widerstand (28) und besagtem langgezogenen flexiblen Verbindungsglied (18) in Reihe elektrisch verbunden ist und daß ein zweites elektrisches Kabel (38) mit besagtem ersten Teil (14) und einem Teil der besagten Steckverbindung (16, 17) verbunden ist und eine größere Länge als besagtes flexibles Verbindungsglied hat, so daß bei gespanntem flexiblem Glied das zweite Kabel ungespannt ist.

2. Vorrichtung nach Anspruch 1, worin das zweite Kabel (38) um das flexible Glied (18) gewunden ist.

3. Vorrichtung nach Anspruch 1 oder 2, worin das flexible Glied (18) eine Metallkette aus aneinandergereihten Perlen und/oder anderen Gliedern besteht.

4. Vorrichtung nach Anspruch 1, 2 oder 3, worin das besagte erste Teil ein Stecker (14) ist, der axial in eine Anschlußbüchse (12) eingesteckt werden kann, wobei besagter Stecker eine axiale Verlängerung (14a) besitzt, auf welcher das flexible Glied (18) mit relativ schwenkbarer Bewegung montiert ist.

5. Vorrichtung nach einem der vorhergehenden Ansprüche, worin das zweite Teil (11a) bandförmig ausgelegt ist und worin besagtes flexibles Mittel lösbare durch einen Schnappkontakt (36a, 36b) auf dem Band und dem ersten erwähnten elektrischen Kabel am zweiten Teil befestigt ist.

## Revendications

1. Un dispositif de sûreté connectable entre une personne humaine et une borne à conduction électrique, le dispositif étant composé d'un premier composant à conductivité électrique (14) connectable à ladite borne (12), un deuxième composant à conductivité électrique (11a) connectable à ladite personne humaine, et des moyens souples électriques assurant le raccordement électrique desdits composants (14, 11a), lesdits moyens de raccordment souples comprenant en série électrique un premier câble électrique (15), une résistance électrique de protection (28) et un member souple rallongé (18), caractérisé par un connecteur relâchable à fiche et prise (16, 17) connecté en série électrique entre ladite résistance électrique (28) et ledit membre souple rallongé de connexion (18), et caractérisé par un deuxième câble électrique (38) relié par des moyens électriques audit premier composant (14) et à l'un desdits prise et fiche (14, 17) et étant d'une longueur supérieure à celle dudit membre de raccordement électrique souple de sorte que lorsque le membre souple est sous tension le deuxième câble est libre de tension.

2. Le dispositif décrit dans la Revendication 1, dans lequel la deuxième câble (38) est enroulé autour du membre souple (18).

3. Le dispositif décrit dans l'une ou l'autre des Revendications 1 et 2, dand lequel le membre souple (18) est une chaîne métallique de boules interconnectées ou d'autres membres de raccordement.

4. Le dispositif décrit dans l'une quelconque des Revendications 1, 2 ou 3 dans lequel ledit premier composant est une fiche (14) axialement insérable dans une prise-borne (12), ladite fiche étant munie d'une rallonge axiale (14a) sur laquelle le membre souple (18) est monté à des fins de déplacement relatif par basculement.

5. Le dispositif décrit dans l'une quelconque des Revendications qui précèdent, dans lequel le deuxième composant (11a) prend la forme d'une lanière et dans lequel ledit moyen souple est attachée d'une manière relâchable au deuxième composant au moyen d'un connecteur à rupture facile (36a, 36b) porté par la lanière et par le premier des câbles électriques mentionnés.

FIG.1

FIG.2

FIG.3